(19) Europäisches Patentamt
European Patent Office
Office européen des brevets

(11) **EP 1 882 738 A1**

(12) **EUROPEAN PATENT APPLICATION**
published in accordance with Art. 153(4) EPC

(43) Date of publication:
**30.01.2008 Bulletin 2008/05**

(51) Int Cl.:
**C12N 15/09** (2006.01)    **C12N 1/02** (2006.01)
**C12N 7/02** (2006.01)    **C12N 11/14** (2006.01)

(21) Application number: **06756399.9**

(22) Date of filing: **19.05.2006**

(86) International application number:
**PCT/JP2006/310043**

(87) International publication number:
**WO 2006/123781 (23.11.2006 Gazette 2006/47)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.05.2005 JP 2005148256
29.07.2005 JP 2005222291
15.12.2005 JP 2005362034**

(71) Applicant: **Arkray, Inc.
Kyoto-shi, Kyoto 601-8045 (JP)**

(72) Inventors:
• **HASHIGUCHI, Satoshi,
c/o Arkray, Inc.
Kyoto-shi, Kyoto 601-8045 (JP)**

• **HIRAI, Mitsuharu,
c/o Arkray, Inc.
Kyoto-shi, Kyoto 601-8045 (JP)**
• **HOSOMI, Toshiya,
c/o Arkray Inc.
Kyoto-shi, Kyoto 601-8045 (JP)**

(74) Representative: **Rutherford, Jodie et al
Frank B. Dehn & Co.
St Bride's House
10 Salisbury Square
GB-London EC4Y 8JD (GB)**

(54) **METHODS FOR RECOVERING MICROORGANISM AND NUCLEIC ACID USING FINE PARTICLE AND KIT TO BE USED FOR THE METHODS**

(57)     The present invention provides a method of collecting microorganisms and a method of collecting nucleic acids, which both can be carried out easily and can achieve a high collection rate. A method of collecting microorganisms according to the present invention includes a microorganism adsorption step of bringing a sample into contact with fine particles so as to cause microorganisms contained in the sample to be adsorbed onto the fine particles. In this method, the fine particles have a particle diameter of 6 μm or less and a specific surface area of 50 m2/g or less. Furthermore, a method of collecting nucleic acids according to the present invention includes: a microorganism adsorption step of causing microorganisms to be adsorbed onto fine particles; and a nucleic acid elution step of eluting nucleic acids from the microorganisms that have been adsorbed onto the fine particles. The microorganism adsorption step in this method is the microorganism collection method of the present invention. According to the collection methods of the present invention, microorganisms and nucleic acids can be collected easily and efficiently. Preferably, the fine particles are magnetic silica particles.

FIG. 9

**Description**

Technical Field

The present invention relates to a method of collecting microorganisms using fine particles, a method of collecting nucleic acids using fine particles, and kits for use in these methods.

Background Art

Nucleic acid analyses play an important role in making genetic level diagnoses of infectious diseases and hereditary diseases in the medical field. Nowadays, nucleic acid analyses are applied and used not only in the medical field but also in various fields such as agriculture and food industry.

To conduct a nucleic acid analysis, it is necessary to collect nucleic acids from a sample (e.g., a germ culture solution, urine, or blood). One example of the method of collecting nucleic acids is a method using fine particles such as magnetic particles (see Patent Document 1, for example). The method disclosed in this document is based on the finding that, since germs bind to magnetic particles non-specifically under acidic conditions, it is possible to separate them together with the magnetic particles by means of magnetic force. More specifically, the method is carried out in the following manner. First, magnetic particles are mixed in a germ culture solution under acidic conditions, thereby fixing germs on the magnetic particles. Thereafter, the magnetic particles are collected from the liquid by applying a magnetic field. Then, the magnetic particles are suspended to separate the germs from the magnetic particles. After that, the germs are dissolved to elute DNAs. Target DNAs can be obtained by fixing the eluted DNAs on the magnetic particles.

Examples of a method of collecting nucleic acids using other fine particles include a method of collecting nucleic acids by fixing cells on solid supports such as magnetic silica particles, then eluting nucleic acids of the cells, and binding the nucleic acids to the solid supports (see Patent Document 2, for example).

These methods have an advantage in that they are adaptable to automation with machinery because they do not require a centrifuging operation for separating germs from a sample. Nevertheless, these methods still have room for improvement because they cannot achieve a sufficient collection rate of germs or nucleic acids from a sample. Furthermore, in order to improve the collection rate of germs or nucleic acids, it is necessary to increase the amount of magnetic particles to be used, which is disadvantageous in terms of cost.

Therefore, there has been great demand for a method of collecting microorganisms and a method of collecting nucleic acids, which both can be carried out easily and can achieve a high collection rate.

Patent Document 1: JP 2003-521228 A
Patent Document 2: JP 2002-507116 A

Disclosure of Invention

Problem to be Solved by the Invention

Therefore, with the foregoing in mind, it is an object of the present invention to provide a method of collecting microorganisms and a method of collecting nucleic acids, which both can be carried out easily and can achieve a high collection rate.

Means for Solving Problem

The present invention provides a method of collecting microorganisms using fine particles. The method includes a microorganism adsorption step of bringing a sample into contact with fine particles so as to cause microorganisms contained in the sample to be adsorbed onto the fine particles. In this method, the fine particles have a particle diameter of 6 $\mu$m or less and a specific surface area of 50 $m^2$/g or less.

The present invention also provides a method of collecting nucleic acids. The method includes: a microorganism adsorption step of causing microorganisms to be adsorbed onto fine particles; and a nucleic acid elution step of eluting nucleic acids from the microorganisms that have been adsorbed onto the fine particles. In this method, the microorganism adsorption step is the microorganism collection method according to the present invention.

Effects of the Invention

The inventors of the present invention conducted a keen study to improve the collection rates of microorganisms and nucleic acids from a sample. In the course of this study, they found that, when collecting microorganisms using fine particles, the particle diameter and the specific surface area of the fine particles to be used gave a significant influence on the collection rate of microorganisms. Based on this finding, the inventors of the present invention conducted a further in-depth study, thereby achieving the present invention. That is, in order to improve the collection rate of microorganisms, one usually may consider using fine particles having a large specific surface area to increase the total surface area of the same. However, it was found that, when fine particles having a large specific surface area were used, microorganisms could not be adsorbed thereon sufficiently even though the total surface area of the fine particles was large. The reason for this presumably is as follows. Examples of the fine particles having a large specific surface area include those having a large number of pores on their surfaces. It is considered that, however, since the contact area between the microorganisms and the fine particles is small in such a case, the microorganisms cannot be adsorbed sufficiently In contrast, in the case of the fine particles having a particle diameter of 6 $\mu$m or less and a specific surface area of 50 $m^2$/g or less as used in the microorganism collection method of the present invention, it is considered that, for example, since the contact area between the microorganisms and the fine particles is large, the microorganisms can be adsorbed sufficiently, thus increasing the amount of the microorganisms adsorbed on each of the fine particles. Thus, according to the microorganism collection method of the present invention, the collection rate of microorganisms can be improved and besides, the microorganisms can be collected easily because a complicated process such as centrifugation is not necessary. Furthermore, since the collection rate can be improved by using the fine particles as described above, the microorganisms can be collected with a high collection rate even when the amount of fine particles used is smaller than in the prior art, for example.

Furthermore, in the nucleic acid collection method of the present invention, microorganisms are collected by the microorganism collection method of the present invention. Therefore, the microorganisms can be collected with a high collection rate, so that nucleic acids can be collected efficiently because the nucleic acids are extracted from the microorganisms that have been collected with such a high collection rate.

Brief Description of Drawings

[FIG. 1] FIG. 1 is a graph showing the measurement results of real-time PCR performed in Example 7 of the present invention.
[FIG. 2] FIG. 2 is a graph showing the measurement results of real-time PCR performed in Example 8-1 of the present invention and Comparative Example 6-1.
[FIG. 3] FIG. 3 is a graph showing the measurement results of real-time PCR performed in Example 8-2 of the present invention and Comparative Example 6-2.
[FIG. 4] FIG. 4 is a graph showing the measurement results of real-time PCR performed in Example 9 of the present invention and Comparative Example 7.
[FIG. 5] FIG. 5 is a graph showing the measurement results of real-time PCR performed in Example 10 of the present invention and Comparative Example 8.
[FIG. 6] FIG. 6 is a graph showing the collection rates obtained in Examples 13-1 and 13-2 of the present invention and Comparative Example 11.
[FIG. 7] FIG. 7 is a graph showing the collection rates obtained in Example 13-3 of the present invention and Comparative Example 12.
[FIG. 8] FIG. 8 is a graph showing the collection rates obtained in Examples 14-1 and 14-2 of the present invention and Comparative Example 13.
[FIG. 9] FIG. 9 is a graph showing the collection rates obtained in Examples 14-3 and 14-4 of the present invention and Comparative Example 14.

Description of the Invention

In the present invention, the upper limit of the particle diameter of the fine particles is, as described above, 6 $\mu$m or less, preferably 4 $\mu$m or less, more preferably 2 $\mu$m or less, still more preferably 1 $\mu$m or less, and particularly preferably 0.4 $\mu$m or less. Although the lower limit thereof is not particularly limited, it preferably is 0.1 $\mu$m or more, for example. When the particle diameter of the fine particles is in such a range, the microorganisms can be collected with a high collection rate as described above. Furthermore, since the microorganisms can be collected with a high collection rate even when the amount of the fine particles is smaller than in the prior art, the method according to the present invention particularly is advantageous in terms of cost. Note here that the particle diameter refers to the length of the longest line that connects one point and another point on the outer periphery of the fine particle, and can be measured by direct observation using a scanning electron microscope (SEM), for example. It is only necessary that, for example, at least 50%, preferably at least 70% of the fine particles used have a particle diameter within the above-described range.

The upper limit of the specific surface area of the fine particles is, as described above, 50 m$^2$/g or less, preferably 30 m$^2$/g or less, and more preferably 20 m$^2$/g or less. The lower limit of the specific surface area is not particularly limited, and preferably is 1 m$^2$/g or more. The specific surface area can be calculated by, for example, the BET (nitrogen adsorption) method standardized as a "silica gel test method" specified in JIS K 1150. It is only necessary that, for example, at least 50%, preferably at least 70% of fine particles to be used have a specific surface area within the above-described range.

The particle diameter and the specific surface area of the fine particles may be determined based on, for example, the type of microorganisms to be collected etc., which will be described later. When the substances to be collected are germs or cells, it is preferable that the fine particles have a particle diameter of 0.1 to 6 $\mu$m and a specific surface area of 1 to 50 m$^2$/g, more preferably a particle diameter of 0.1 to 2 $\mu$m and a specific surface area of 1 to 20 m$^2$/g, for example. When the substances to be collected are viruses, it is preferable that the fine particles have a particle diameter of 0.1 to 6 $\mu$m and a specific surface area of 1 to 50 m$^2$/g, more preferably a particle diameter of 0.1 to 2 $\mu$m and a specific surface area of 1 to 30 m$^2$/g, for example.

As the fine particles, it is preferable to use fine particles having a hydroxyl group or the like on their surfaces, for example. Among these, it is preferable to use fine particles having on their surfaces a silica compound or a resin having a hydroxyl group, such as poly(ethlene, alkyl-OH, acrylate) and polyethylene glycol. With the use of such fine particles, microorganisms for example, can be adsorbed thereon still more easily, so that the collection rate can be improved still further.

The fine particles may be magnetic fine particles or non-magnetic fine particles. However, it is preferable to use magnetic particles because, for example, separation of the fine particles and the liquid can be achieved still more easily Note here that it is not necessary that the magnetic particle be entirely magnetic and may be only partially magnetic. That is, the magnetic particle may be, for example, a particle made of a magnetic material, whose surface is at least partially coated with a non-magnetic material or a particle obtained by mixing a magnetic material and a non-magnetic material and then granulating the mixture. Also, commercially available fine particles can be used as the fine particles.

The magnetic material is not particularly limited as long as it is magnetic, and examples thereof include: metals such as iron, chromium, and nickel; metal oxides such as iron oxides and chromium oxides; and magnetic alloys. Examples of the iron oxides include Fe$_3$O$_4$ (magnetite), Fe$_2$O$_3$ (maghemite), and rFe$_2$O$_3$ (r-type ferric oxide). Examples of the non-magnetic material include silica compounds and resins having a hydroxyl group. Examples of the silica compound include glass, Celite diatomaceous earth, silica polymers, magnesium silicate, silicone nitrogen compounds (e.g., SiN$_4$), aluminum silicate, and silicon dioxide. Among these, glass, Celite diatomaceous earth, silica polymers, SiN$_4$, and silicon dioxide are preferable, and glass, Celite diatomaceous earth, SiN$_4$, and silicon dioxide are more preferable. Examples of the resin having a hydroxyl group include poly(ethylene, alkyl-OH, acrylate) and polyethylene glycol.

Among these, the fine particles preferably are magnetic silica particles containing a magnetic material such as a magnetic metal or a magnetic metal oxide, more preferably magnetic silica particles obtained by coating the magnetic material with a silica compound.

Furthermore, examples of the non-magnetic fine particles include particles made of a non-magnetic material, and examples of the non-magnetic material are the same as those described above.

Moreover, since the fine particles used in the present invention can achieve an excellent collection rate as described above, the microorganisms can be collected with a high efficiency even when the amount of the fine particles is smaller than in the prior art. When the amount of the fine particles used can be decreased as described above, it becomes possible to separate the fine particles sufficiently from the liquid from which the microorganisms have been collected, for example. More specifically, in the prior art, it is necessary to increase the amount of fine particles to be used in order to improve the collection rate as described above, which, however, makes it difficult to separate the fine particles sufficiently from the liquid used for collecting microorganisms or nucleic acids. In contrast, in the present invention, since the amount of the fine particles used can be decreased by the use of the above-described fine particles, it becomes possible to separate the fine particles sufficiently from the liquid after the collection. Moreover, in the case where the fine particles cannot be separated sufficiently as in the prior art, the fine particles may remain in a microorganism collection solution when collecting nucleic acids therefrom in the manner as will be described later. When the fine particles remain as described above, the remaining fine particles may serve as reaction inhibitors in, for example, the amplification of the nucleic acids by a PCR (Polymerase Chain Reaction) method, resulting in insufficient amplification of the nucleic acids. Furthermore, when detecting nucleic acids with measuring labels attached thereto by an optical method, measurement error may be caused by the remaining fine particles present in the collection solution. However, according to the present invention, since the microorganisms and the fine particles are separated sufficiently as described above, it becomes possible to avoid the above-described problems caused by the remaining fine particles, for example. Accordingly, for example, a decrease in efficiency in PCR amplification or degradation in analytical accuracy also can be suppressed.

Furthermore, according to the microorganism collection method of the present invention, since fine particles smaller than in the prior art (particle diameter: 6 $\mu$m or less) are used, microorganisms can be collected with a high collection rate even when a sample contains a lot of impurities, for example. Moreover, since the fine particles have a small particle diameter as described above, a larger amount of fine particles than in the prior art can be added to the sample, which allows the collection rate to be improved still further.

In the present invention, the microorganisms include germs, viruses, and cells. The germs are not particularly limited and include bacteria and fungi, and examples thereof include gonococci, chlamydiae, acid-fast bacteria, atypical mycobacteria, *Legionella* bacteria, mycoplasmas, spirochetes, syphilis spirochetes, rickettsiae, *Mycobacterium leprae, Spirillum minus, staphylococci,* streptococci, *Escherichia coli, Pseudomonas aeruginosa,* and *Yersinia pestis.* Examples of the viruses include lambda phage, immunodeficiency viruses, leukemia viruses, Japanese encephalitis viruses, hepatitis B viruses (HBV), hepatitis C viruses (HCV), adult T-cell leukemia viruses (ATLV), human immunodeficiency viruses (HIV), and Ebola viruses. Examples of the cells include leukocytes, epithelial cells, mucosal cells, somatic cells, and other cells derived from animals and plants. There is no particular limitation on the sample, and examples thereof include biological samples, environmental samples collected from domestic waste water, industrial liquid waste, and the like, and chemical samples to be used in chemical analyses. Examples of the biological sample include whole blood, lymph, urine, saliva, sputum, and nasal secretion.

The microorganism collection method of the present invention is, as described above, a method of collecting microorganisms using fine particles, including a microorganism adsorption step of bringing a sample into contact with fine particles so as to cause microorganisms contained in the sample to be adsorbed onto the fine particles. In this method, the fine particles have a particle diameter of 6 $\mu$m or less and a specific surface area of 50 m$^2$/g or less.

In the microorganism collection method of the present invention, it is preferable that a microorganism collection solution containing the fine particles is provided beforehand, and the sample is brought into contact with the microorganism collection solution in the microorganism adsorption step, thus causing the microorganisms in the sample to be adsorbed onto the fine particles. The content of the fine particles in the microorganism collection solution is not particularly limited, and can be determined based on the type and the amount of the microorganisms to be collected, the particle diameter and the specific surface area of the fine particles to be used, etc, for example. When the sample is urine, blood, or the like, it is preferable to use, for example, 0.1 to 10 mg of fine particles, more preferably 1 to 5 mg of fine particles, with respect to 100 $\mu$l of the sample. It is to be noted that, in the present invention, the microorganism collection solution refers to a reagent used for causing the microorganisms to be adsorbed onto the fine particles.

Preferably, the microorganism collection solution is an acid solution or a neutral solution, for example. By using such a solution, it is possible to achieve a still higher collection rate with the use of a still smaller amount of the fine particles. This is highly advantageous in terms of cost. Preferably, the acid solution has a pH of 2 to 3, for example. When the acid solution is a buffer, examples thereof include a formate buffer, an acetate buffer, a citrate buffer, and a glycine-HCl buffer. When the neutral solution is a buffer, examples thereof include a Tris-HCl buffer. It is preferable that the neutral solution contains a salt such as a Mg salt, a Na salt, a Ca salt, or a K salt, more preferably a Mg salt, for example. These salts may be used alone or in combinations of two or more kinds thereof. By using the neutral solution containing such a salt(s), it is possible to achieve a still higher collection rate with the use of a still smaller amount of the fine particles. Specific examples of the salt include MgC1$_2$, NaCl, CaCl, and KCl, among which MgCl$_2$ is preferable. The concentration of the salt in the neutral solution is 0.25 to 2.0 M, preferably 0.5 to 1.0 M, for example.

It is preferable that the microorganism collection solution contains a protein denaturant, for example. The reason for this is that this further improves the collection rate of microorganisms, thus allowing the microorganisms to be collected with a very high collection rate with the use of a still smaller amount of the fine particles. As the protein denaturant, it is preferable to use a non-surfactant denaturant, for example. Examples of the non-surfactant denaturant include heavy metal salts, organic solvents, and urea. Specific examples of preferable non-surfactant denaturants include guanidium salt, ethanol, and acetic acid.

The fine particles on which the microorganisms have been adsorbed can be collected, for example, with the use of a magnet or the like, as will be described later. Furthermore, separation of the fine particles and the microorganisms adsorbed thereon can be achieved by, for example, suspending the fine particles in a physiological saline or a surfactant that is in a suitable concentration.

Next, as described above, the nucleic acid collection method of the present invention includes a microorganism adsorption step of causing microorganisms to be adsorbed onto the fine particles, which is performed by the microorganism collection method of the present invention; and a nucleic acid elution step of eluting nucleic acids from the microorganisms that have been adsorbed onto the fine particles.

It is preferable that, in the nucleic acid elution step, the nucleic acids are eluted from the microorganisms that have been adsorbed onto the fine particles by bringing the fine particles into contact with a nucleic acid-extraction reagent. The nucleic acid-extraction reagent can be determined in accordance with the type of the microorganisms to be collected etc. The nucleic acid-extraction reagent is not particularly limited, and examples thereof include surfactants such as polyoxyethylene-p-t-octylphenyl ether (e.g., a Triton series surfactant), polyoxyethylene sorbitan alkyl ester (e.g., a Tween series surfactant), and sodium dodecyl sulfate (SDS), and ethylenediaminetetraacetic acid (EDTA). Specific examples of the Triton series surfactant include Triton X-100 (trade name), and specific examples of the Tween series surfactant include Tween 20 (trade name). The solvent of the nucleic acid-extraction reagent is not particularly limited, and can be a buffer such as a Tris-HCl buffer, for example.

The nucleic acid collection method of the present invention further may include a liquid separation step. In this case, it is preferable that, in the microorganism adsorption step, the sample containing the microorganisms is brought into contact with the microorganism collection solution containing the fine particles, thereby causing the microorganisms to be adsorbed onto the fine particles, and then, in the liquid separation step, the fine particles and the liquid (e.g., the microorganism collection solution) are separated, followed by the nucleic acid elution step. The microorganism collection solution is as described above.

As one example of the nucleic acid collection method of the present invention, a method of collecting nucleic acids from microorganisms adsorbed on fine particles will be described.

When the fine particles are magnetic particles, the method can be carried out in the following manner, for example. First, in the liquid separation step, magnetic force is applied from the outside of a container by means of a magnet or the like so that the magnetic particles are adhered to the inner surface of the container. In this state, liquid is removed from the container, and the magnetic particles on which the microorganisms are adsorbed are collected. According to this method, centrifugation for separating unnecessary components (e.g., liquid components) and necessary components (e.g., solid components such as magnetic particles) need not be performed, thus allowing the operation for collecting microorganisms from the sample to be simplified.

Next, in the nucleic acid elution step, the magnetic particles are dispersed in a nucleic acid-extraction reagent and heated, for example. The heating temperature and the heating time are not particularly limited and can be set depending on, for example, the types and the amounts of the microorganisms to be collected and the nucleic acid-extraction reagent. Preferably, the heating temperature is 80°C to 100°C and the heating time is 1 to 10 minutes, for example.

Then, in the same manner as in the liquid separation step, magnetic force is applied from the outside of the container so that the magnetic particles are adhered to the inner surface of the container. In this state, the nucleic acid-extraction reagent that contains nucleic acids is collected from the container. In this manner, the nucleic acids can be collected. According to this method, centrifugation for separating unnecessary components (e.g., magnetic particles) and necessary components (the nucleic acid-extraction reagent that contains the nucleic acids) need not be performed, thus allowing the operation for collecting nucleic acids to be simplified.

On the other hand, when the fine particles are not magnetic particles, the collection can be carried out in the following manner, for example. In the liquid separation step, for example, the fine particles are allowed to settle naturally by their own weight, and the fine particles and the liquid are separated. Then, nucleic acids are eluted by performing the nucleic acid elution step in the same manner as that in the case of the magnetic particles. Subsequently, the fine particles are allowed to settle naturally by their own weight as in the liquid separation step, for example, and thereafter, a supernatant containing the nucleic acids is collected. In this manner, the nucleic acids can be collected.

Next, a kit for collecting microorganisms according to the present invention is a kit to be used in the microorganism collection method of the present invention. The kit includes fine particles having a particle diameter of 6 $\mu$m or less and a specific surface area of 50 m$^2$/g or less. According to the kit of the present invention, microorganisms can be collected with a high collection rate with the use of such fine particles. Furthermore, since a high collection rate can be achieved even when the amount of the fine particles is smaller than in the prior art, the kit according to the present invention is highly advantageous in terms of cost. As the fine particles, the same fine particles as those usable in the microorganism collection method of the present invention can be used, and among these fine particles, magnetic silica particles are preferable. The methods of determining the particle diameter and the specific surface area are as described above.

It is preferable that the kit for collecting microorganisms according to the present invention further includes a microorganism collection solution containing the fine particles. Examples of the microorganism collection solution include those usable in the microorganism collection method of the present invention.

Next, a kit for collecting nucleic acids according to the present invention is a kit to be used in the nucleic acid collection method of the present invention. The kit includes: fine particles having a particle diameter of 6 $\mu$m or less and a specific surface area of 50 m$^2$/g or less; and a nucleic acid-extraction reagent. According to the kit of the present invention, nucleic acids can be collected with a high collection rate with the use of such fine particles. Furthermore, since a high collection rate can be achieved even when the amount of the fine particles is smaller than in the prior art, the kit according to the present invention is highly advantageous in terms of cost. As the fine particles, the same fine particles as those usable in the microorganism collection method of the present invention can be used, and among these fine particles, magnetic silica particles coated with a silica compound are preferable. Examples of the nucleic acid-extraction reagent include those usable in the nucleic acid collection method of the present invention. The methods of determining the particle diameter and the specific surface area are as described above.

Preferably, the kit for collecting nucleic acids according to the present invention further includes a microorganism collection solution containing the fine particles. Examples of the microorganism collection solution include those usable in the microorganism collection method of the present invention.

Hereinafter, examples of the present invention will be described along with comparative examples. It is to be noted, however, that the present invention is by no means limited to the examples and comparative examples given below. In the following examples and comparative examples, the particle diameter refers to the length of the longest line connecting one point and another point on the outer periphery of a fine particle, which is determined by an electron microscope, and the specific surface area is determined based on the BET (nitrogen adsorption) method standardized by the "silica gel test method" specified in JIS K 1150.

Example 1

The present example is an example where germs (gonococci) were collected using magnetic silica particles under acidic conditions, and nucleic acids were collected from the thus-collected germs.

(Collection of germs under acidic conditions)

First, magnetic silica particle-containing solutions (200 mg/ml) were prepared using respective types of magnetic silica particles shown in Table 1 below. Then, 20 $\mu$l of each of the magnetic silica particle-containing solutions was mixed with 100 $\mu$l of 0.5 M glycine-HCl (pH 3), thus preparing a microorganism collection solution.

[Table 1]

| | | Particle diameter ($\mu$m) | Specific surface area (m$^2$/g) |
|---|---|---|---|
| Ex. 1-1 | Trade name: MT03v2-r1 (BANDO CHEMICAL INDUSTRIES; LTD.) | 0.1 to 0.4 | 2 to 10 |
| Ex. 1-2 | Trade name: Micromer(R)-M (Micromod) | 2 | 2.7 |
| Ex. 1-3 | Trade name: MagExtractor(R)- Genome (TOYOBO CO., LTD.) | 2 to 6 | 7.06 |
| Comp. Ex. 1 | Trade name: Micromer(R)-M (Micromod) | 12 | 0.45 |

In Table 1, the fine particles used in Examples 1-1 and 1-2 and Comparative Example 1 were surface-modified with $SiO_2$. The fine particles used in Example 1-3 were magnetic silica particles included in a gene extraction kit available under the trade name "MagExtractor (R)-Genome".

Next, gonococcus colonies cultured in a chocolate agar medium (Nissui Pharmaceutical Co., Ltd.) were collected and then suspended in a physiological saline to prepare a germ solution whose absorbance at the wavelength of 530 nm ($OD_{530}$) was 0.18.

The germ solution was diluted with a physiological saline to 100 times the original volume. Then, 100 $\mu$l of this diluted germ solution was mixed with 120 $\mu$l of the above-described microorganism collection solution, and the germs were adsorbed onto the magnetic silica particles by subjecting the resultant mixture to pipetting 10 times. With the magnetic silica particles being attracted to a magnet, the supernatant was removed. Thereafter, 200 $\mu$l of 10 mM glycine-HCl (pH 3) was added and the resultant mixture was subjected to pipetting 10 times, after which the supernatant was removed in the same manner as described above. In this manner, the magnetic silica particles were washed. This washing operation was repeated to a total of three times. Impurities were thus removed, and the germs contained in the germ solution were collected in the state of being adsorbed onto the magnetic silica particles.

(Collection of nucleic acids from germs)

To the collected magnetic silica particles, 100 $\mu$l of a nucleic acid-extraction reagent was added. Nucleic acids were extracted from the germs by subjecting the resultant mixture to pipetting 10 times, heating the mixture at 95°C for 5 minutes, and further subjecting the mixture to pipetting 10 times. With the magnetic silica particles being attracted to a magnet, an extract of the nucleic acids was collected. As the nucleic acid-extraction reagent, a mixed solution of 10 mM Tris-HCl (pH 8), 0.1 mM ethylenediaminetetraacetic acid (EDTA) (pH 8), and 1 wt% Triton X-100 (trade name) was used.

(Calculation of nucleic acid collection rate)

Using reagents shown in Table 2 below and i-Cycler™ (trade name, Bio-Rad Laboratories), the extracted nucleic acids were amplified by PCR, which was performed by heating the extracted nucleic acids at 50°C for 2 minutes and at 95°C for 2 minutes, followed by 50 cycles each of which consisted of 95°C at 10 seconds and 56°C at 60 seconds. The extracted nucleic acids were amplified by PCR, during which the fluorescence intensity was measured in real time. The Ct value (Ct 1) of the nucleic acids was determined by measuring the cycle number at which the fluorescence intensity reached 100. The measurement was performed 6 times with regard to each of the examples and comparative example. Note here that the amount of each reagent shown in Table 2 below is the amount of the reagent to be used with respect to 1.5 μl of the extract of the nucleic acids.

[Table 2]

| H$_2$O | 18.65 μl |
|---|---|
| 10 × GeneTaq Buffer (trade name, NIPPON GENE CO., LTD.) | 2.5 μl |
| 10 mM AUGC | 0.5 μl |
| 100 mM MgCl$_2$ | 0.375 μl |
| 5 μM *F-D-NG-R1-32 | 1 μl |
| 100 μM *NG-F3405-20 | 0.125 μl |
| 100 μM *NG-3526-20R | 0.125 μl |
| 2 U/μl UNG (TREVIGEN, Inc) | 0.1 μl |
| 5 U/μl GeneTaq NT (trade name, NIPPON GENE CO., LTD.) | 0.125 μl |
| | 23.5 μl |

*F-D-NG-R1-32: (FAM)-acttagagacgttacggaaaaatatcaacgag-(DABCYL) [SEQ ID NO: 1]
*NG-F3405-20: 5'-gcggttattttctgctcgct-3' [SEQ ID NO: 2]
*NG-3526-20R: 5'-accttcgagcagacatcacg-3' [SEQ ID NO: 3]
Note here that the above-noted AUGC contained ATP, GTP, and CTP (all available from Takara) and UTP (Roche).

On the other hand, as a control, 1 μl of the above-described germ solution exhibiting OD$_{530}$ of 0.18 was added to 100 ul of the above-described nucleic acid-extraction reagent and the resultant mixture was heated at 95°C for 5 minutes to lyse the germs, thereby extracting nucleic acids. The nucleic acids then were amplified by PCR, during which the fluorescence intensity was measured in real time. The Ct value (Ct 2) was determined by measuring the cycle number at which the fluorescence intensity reached 100. This measurement was performed 6 times.
The collection rate of the nucleic acids was calculated based on the following equation, using the Ct value (Ct1) obtained in each of the example or the comparative example and the Ct value (Ct2) obtained in the control. The thus-obtained Ct values and collection rates of the nucleic acids (each denotes an average value) are shown in Table 3 below.

$$\text{Collection rate (\%)} = 100/2^{(Ct2-Ct1)}$$

[Table 3]

| | Particle diameter (μm) | Specific surface area (m$^2$/g) | Ct value | Collection rate of nucleic acids (%) |
|---|---|---|---|---|
| Ex. 1-1 | 0.1 to 0.4 | 2 to 10 | 25.9 | 57 |
| Ex. 1-2 | 2 | 2.7 | 25.7 | 66 |
| Ex. 1-3 | 2 to 6 | 7.06 | 25.8 | 62 |
| Comp. Ex. 1 | 12 | 0.45 | 27.6 | 18 |

(continued)

|  | Particle diameter (μm) | Specific surface area (m²/g) | Ct value | Collection rate of nucleic acids (%) |
|---|---|---|---|---|
| Control | - | - | 25.1 | 100 |

As shown in Table 3, in Examples (1-1, 1-2, and 1-3) where the fine particles having a particle diameter of 6 μm or less and a specific surface area of 50 m²/g or less were used, the nucleic acids could be collected with a high collection rate. On the other hand, in Comparative Example 1 where the fine particles having a particle diameter not satisfying the above-described range were used, the collection rate was very low and the collection of the nucleic acids was insufficient. From these results, it can be said that, with the use of fine particles having a particle diameter of 6 μm or less and a specific surface area of 50 m²/g or less, it is possible to collect nucleic acids (germs) efficiently.

Example 2

Nucleic acids were collected in the same manner as in Example 1, except that the amount of the magnetic silica particle-containing solution (40 mg/ml) added was set to 10 μl so that the amount of silica particles used became 1/10 of that in Example 1. The results are shown in Table 4 below.
[Table 4]

|  | Particle diameter (μm) | Specific surface area (m²/g) | Ct value | Collection rate of nucleic acids (%) |
|---|---|---|---|---|
| Ex. 2-1 | 0.1 to 0.4 | 2 to 10 | 26.0 | 44 |
| Ex. 2-2 | 2 | 2.7 | 25.6 | 57 |
| Ex. 2-3 | 2 to 6 | 7.06 | 26.6 | 29 |
| Comp. Ex. 2 | 12 | 0.45 | 28.3 | 9 |
| Control | - | - | 24.8 | 100 |

As shown in Table 4, in Examples (2-1, 2-2, and 2-3) where the fine particles having a particle diameter of 6 μm or less and a specific surface area of 50 m²/g or less were used, the nucleic acids could be collected with a high collection rate even though the amount of the magnetic silica particles was small (1/10 of that in Example 1). In contrast, in Comparative Example 2 where the fine particles having a particle diameter not satisfying the above-described range were used, the collection rate was very low and the collection of the nucleic acids was insufficient. From these results, it can be said that, with the use of fine particles having a particle diameter of 6 μm or less and a specific surface area of 50 m²/g or less, it is possible to collect nucleic acids (germs) efficiently even when the amount of the magnetic silica particles is small.

Example 3

The present example is an example where germs (gonococci) were collected using a neutral solution containing magnetic silica particles and a salt, and nucleic acids were collected from the thus-collected germs.

(Collection of germs under neutral conditions)

First, germ solutions and microorganism collection solutions were prepared. More specifically, they were prepared in the following manner. First, six types of buffers, namely, 0.5 M Tris-HCl buffers (pH 7.1) respectively containing five types of salts shown in Table 6 below (concentration: 0.5 M) and a buffer containing no salt, were prepared. Then, 100 μl of each of the above-described buffers was mixed with 10 μl of each of magnetic silica-containing solutions respectively containing magnetic silica particles shown in Table 5 below (400 mg/ml). Thus, the microorganism collection solutions were prepared. On the other hand, the germ solutions were prepared by diluting gonococcus suspensions (OD$_{530}$: 0.18) prepared in the same manner as in Example 1 with a physiological saline to 100 times the original volumes.
[Table 5]

| | | Particle diameter ($\mu$m) | Specific surface area (m²/g) |
|---|---|---|---|
| Ex. 3-1 | Trade name: MT03v2-r1 (BANDO CHEMICAL INDUSTRIES, LTD.) | 0.1 to 0.4 | 2 to 10 |
| Ex. 3-2 | Trade name: Micromer(R)-M (Micromod) | 2 | 2.7 |
| Comp. Ex. 3 | Trade name: Micromer(R)-M (Micromod) | 12 | 0.45 |

Next, 110 $\mu$l of the microorganism collection solution was mixed with 100 $\mu$l of the diluted germ solution, and the germs were adsorbed onto the magnetic silica particles by subjecting the resultant mixture to pipetting for 1 minute. Thereafter, a supernatant was removed in the same manner as described above. Subsequently, 200 $\mu$l of distilled water was added and the resultant mixture was subjected to pipetting 10 times, after which the supernatant was removed in the same manner as described above. In this manner, the magnetic silica particles were washed. This washing operation was repeated to a total of three times. Impurities were thus removed, and the germs contained in the germ solution were collected in the state of being adsorbed onto the magnetic silica particles.

(Calculation of nucleic acid collection rate)

Extraction of nucleic acids and PCR were performed in the same manner as in Example 1, and the Ct values were determined and the collection rates (%) of the nucleic acids were calculated. The Ct values and the collection rates of the nucleic acids obtained are shown in Table 6 below. Note here that each of the values shown in Table 6 is an average value obtained after performing the measurement six times, and the value in parentheses is the Ct value. Furthermore, the Ct values (Ct 2) of controls used for calculating the collection rates were: 24.0 in Example 3-1, and 22.2 in Example 3-2 and Comparative Example 3.
[Table 6]

| | None | LiCl | KCl | NaCl | MgCl$_2$ | CaCl$_2$ |
|---|---|---|---|---|---|---|
| Ex. 3-1 | 16 (26.6) | 14 (26.8) | 19 (26.4) | 22 (26.2) | 54 (24.9) | 22 (26.2) |
| Ex. 3-2 | 18 (24.9) | 13 (25.1) | 14 (25.0) | 13 (25.2) | 20 (24.5) | 33 (23.8) |
| Comp. Ex. 3 | 8 (25.9) | 6 (26.2) | 4(26.8) | 5 (26.4) | 11 (25.4) | 4 (25.4) |

As shown in Table 6, in Examples (3-1 and 3-2) where the fine particles having a particle diameter of 6 $\mu$m or less and a specific surface area of 50 m²/g or less were used, the microorganisms could be collected with a higher collection rate than in Comparative Example 3 where the fine particles having a particle diameter not satisfying the above-described range were used, regardless of the types of the buffers.

Example 4

The present example is an example where viruses (HBVs) in plasma were collected using a buffer containing magnetic silica particles and a protein denaturant, and nucleic acids were collected from the viruses.

(Collection of viruses)

First, using magnetic silica particles having a particle diameter of 0.1 to 0.4 $\mu$m and a specific surface area of 2 to 10 m²/g (BANDO CHEMICAL INDUSTRIES, LTD., trade name: S-M-03), a magnetic silica particle-containing solution (500 mg/ml) was prepared. 1.0 $\mu$l of HBV Plasma (ProMedDx, "Number 10222136") was added to 100 $\mu$l of plasma collected from a healthy subject. Then, to the resultant mixture, any one of buffers shown in Table 8 below and 16 $\mu$l of the magnetic silica particle-containing solution were added. Subsequently, viruses were adsorbed onto the magnetic silica particles by subjecting the resultant mixture to pipetting 30 times, and the supernatant was removed in the same manner as described above. 100 $\mu$l of 10 mM glycine-HCl (pH 3) was added to the magnetic silica particles on which the viruses were adsorbed. The resultant mixture was subjected to pipetting 10 times, after which the supernatant was removed in the same manner as described above. In this manner, the magnetic silica particles were washed. This washing operation was repeated to a total of three times.

(Collection of nucleic acids)

100 µl of a nucleic acid-extraction reagent was added to the washed magnetic silica particles. Nucleic acids were extracted from the viruses by subjecting the resultant mixture to pipetting 10 times, heating the mixture at 95°C for 5 minutes, and further subjecting the mixture to pipetting 10 times. An extract of the nucleic acids was collected in the same manner as described above. As the nucleic acid-extraction reagent, a mixed reagent of 10 mM Tris-HCl (pH 8), 0.1 mM EDTA (pH 8), and 0.1 wt% SDS was used.

As a control, nucleic acids of HBV were extracted by adding 100 µl of the nucleic acid-extraction reagent to 1 µl of the HBV Plasma (ProMedDx, "Number10222136"), subjecting the resultant mixture to pipetting 10 times, heating the mixture at 95°C for 5 minutes, and then further subjecting the mixture to pipetting 10 times.

(Calculation of nucleic acid collection rate)

Using the extract (1.0 µl) of the nucleic acids, reagents shown in Table 7 below, and i-Cycler™ (trade name, Bio-Rad Laboratories), PCR amplification was performed by heating the extracted nucleic acids at 50°C for 2 minutes and 95°C for 2 minutes, followed by 50 cycles each of which consisted of 95°C for 30 seconds and 56°C for 60 seconds, and the Ct value was determined by measuring the cycle number at which the fluorescence intensity reached 250. The measurement was performed 3 times with regard to each of the examples and comparative example.

[Table 7]

| | |
|---|---|
| $H_2O$ | 19.865 µl |
| 10 × GeneTaq Buffer (trade name, NIPPON GENE CO., LTD.) | 2.5 µl |
| 10 mM AUGC | 0.5 µl |
| 7 µM *Taqman-B-F-1-32 | 0.71 µl |
| 100 µM *HBV F50-74 | 0.125 µl |
| 100 µM *HBV R136-109 | 0.125 µl |
| 2 U/µl UNG(TREVIGEN, Inc) | 0.05 µl |
| 5 U/µl GeneTaq NT (trade name, NIPPON GENE CO., LTD.) | 0.125 µl |
| | 24 µl |

*Taqman-B-F-1-32: (FAM)-caacaaccgaccttgaggcatacttcaaagac-(TAMRA) [SEQ ID NO: 4]
*HBV F50-74: 5'-gaggactcttggactctcagcaatg-3' [SEQ ID NO: 5]
*HBV R136-109: 5'-cccaactcctcccagtctttaaacaaac-3' [SEQ ID NO: 6]

The collection rates were calculated based on the Ct values in the same manner as in Example 1. The results are shown in Table 8 below. Note here that, in Table 8, each of the collection rates of the nucleic acids is an average value obtained after the three measurements.

[Table 8]

| | Buffer | | Collection rate of nucleic acids (%) |
|---|---|---|---|
| | Type | Added amount (µl) | |
| Ex. 4-1 | 0.5 M glycine-HCl (pH 3) 2.5 M guanidine hydrochloride | 100 | 57 |
| Ex. 4-2 | 0.5 M glycine-HCl (pH 3) 40% ethanol | 100 | 87 |
| Ex. 4-3 | acetic acid | 25 | 81 |
| Control | - | - | 100 |

As shown in Table 8, the nucleic acid could be collected with a high collection rate in all the examples. In particular, in the case where the glycine-HCl buffer containing ethanol was used (Example 4-2), the collection rate achieved was very high.

Example 5

The present example is another example where viruses (HBVs) in plasma were collected using magnetic silica particles, and nucleic acids were collected from the viruses.

The virus collection, the nucleic acid extraction, and the calculation of the collection rate of nucleic acids were carried out in the same manner as in Example 4, except that a 0.5 M glycine-HCl buffer (pH 3) containing 2.5 M guanidine hydrochloric acid was used as a buffer for collecting microorganisms and fine particles shown in Table 9 below were used. The collection rates of the nucleic acids obtained are shown in Table 9 below together with the result of a control.

[Table 9]

|  |  | Particle diameter ($\mu$m) | Specific surface area ($m^2$/g) | Collection rate of nucleic acids (%) |
|---|---|---|---|---|
| Ex. 5-1 | Trade name: MT03v2-rl (BANDO CHEMICAL INDUSTRIES, LTD.) | 0.1 to 0.4 | 2 to 10 | 76 |
| Ex. 5-2 | Trade name: Micromer[R]-M (Micromod) | 2 | 2.7 | 81 |
| Comp. Ex. 4 | Trade name: Micromer[R]-M (Micromod) | 12 | 0.45 | 23 |
| Control | - | - | - | 100 |

As shown in Table 9, in the Examples (5-1 and 5-2) where fine particles having a particle diameter of 6 $\mu$m or less and a specific surface area of 50 $m^2$/g or less were used, the nucleic acid could be collected with a high collection rate. On the other hand, in Comparative Example 4 where the fine particles having a particle diameter not satisfying the above-described range were used, the collection rate was low and the collection of the nucleic acids was insufficient.

Example 6

The present example is still another example where viruses (HBVs) in plasma were collected using magnetic silica particles, and nucleic acids were collected from the viruses.

The virus collection, the nucleic acid extraction, and the calculation of the collection rate of nucleic acids were carried out in the same manner as in Example 5, except that, a 0.5 M glycine-HCl buffer (pH 3) containing 40% ethanol was used as a buffer for collecting microorganisms. The collection rates of the nucleic acids obtained are shown in Table 10 below together with the result of a control.

[Table 10]

|  | Particle diameter ($\mu$m) | Specific surface area ($m^2$/g) | Collection rate of nucleic acids (%) |
|---|---|---|---|
| Ex. 6-1 | 0.1 to 0.4 | 2 to 10 | 71 |
| Ex. 6-2 | 2 | 2.7 | 81 |
| Comp. Ex. 5 | 12 | 0.45 | 31 |
| Control | - | - | 100 |

As shown in Table 10, in Examples (6-1 and 6-2) where the fine particles having a particle diameter of 6 $\mu$m or less and a specific surface area of 50 $m^2$/g or less were used, the collection rate of nucleic acids was high. On the other hand, in Comparative Example 5 where the fine particles having a particle diameter not satisfying the above-described range were used, the collection rate was low and the collection of the nucleic acids was insufficient.

Example 7

The present example is an example where cells (leukocytes) were collected using magnetic silica particles, and nucleic acids were collected from the cells.

(Collection of leukocytes)

50 µl of heparin blood was added to 500 µl of a physiological saline. Then, to the resultant mixture, 100 µl of any one of the buffers shown in Table 11 and 5 µl of a magnetic silica particle-containing solution (500 mg/ml) were added. Subsequently, the thus-obtained mixture was stirred in a vortex for 1 minute so that cells (leukocytes) were adsorbed onto the magnetic silica particles. A supernatant was removed in the same manner as described above, and the magnetic silica particles on which the cells (leukocytes) were adsorbed were collected. As the magnetic silica particles, magnetic silica particles available under the trade name S-M-03 (BANDO CHEMICAL INDUSTRIES, LTD., particle diameter: 0.1 to 0.4 µm, specific surface area: 2 to 10 $m^2$/g, surface-modified with $SiO_2$) were used.

500 µl of a cleaning solution shown in Table 11 below as corresponding to the above-described buffer was added to the magnetic silica particles. The cleaning solution was stirred for 5 seconds using a vortex, and the cleaning solution then was removed in the same manner as described above. In this manner, the magnetic silica particles were washed. This washing operation was repeated to a total of three times.

[Table 11]

|  | Buffer | Cleaning solution |
|---|---|---|
| Ex. 7-1 | 0.5 M glycine-HCll (pH 3) | 10 mM glycine-HCl (pH 3) |
| Ex. 7-2 | 0.5 M Tris-HCl (pH 7.2) 1 M $MgCl_2$ | 10 mM Tris-HCl (pH 8) 0.1 mM EDTA (pH 8) |

(Collection of nucleic acids)

To the magnetic silica particles on which the leukocytes were adsorbed, 50 µl of a nucleic acid-extraction reagent was added. Nucleic acids were extracted from the leukocytes by stirring the resultant mixture for 5 seconds in a vortex, heating the mixture at 95°C for 5 minutes, and further stirring the mixture for 5 seconds in the vortex. An extract of the nucleic acids was collected in the same manner as described above. As the nucleic acid-extraction reagent, a mixed reagent of 10 mM Tris-HCl (pH 8), 0.1 mM EDTA (pH 8), and 1% Triton X-100 (trade name) was used.

(Nucleic acid-collecting ability)

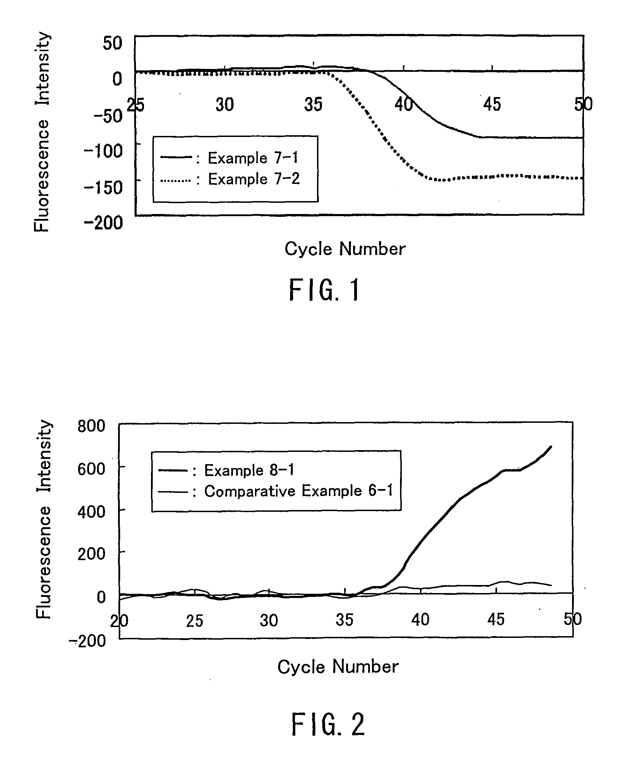

Using the extract (1.0 µl) of the nucleic acids, reagents shown in Table 12 below, and i-Cycler™ (trade name, Bio-Rad Laboratories), PCR amplification was performed by heating the extracted nucleic acids at 95°C for 1 minute, followed by 50 cycles each of which consisted of 90°C for 1 second and 56°C for 15 seconds. The change in fluorescence intensity caused by the PCR amplification was measured over time. The results obtained are shown in FIG. 1.

[Table 12]

| | |
|---|---|
| $H_2O$ | 40.75 µl |
| 10 × GeneTaq Buffer (trade name, NIPPON GENE CO., LTD.) | 2.5 µl |
| 2.5 mM dNTPs (NIPPON GENE CO., LTD.) | 4.0 µl |
| 100 mM $MgCl_2$ | 0.5 µl |
| 5 µM *3FL-27-wt-F3-27 | 0.25 µl |
| 100 µM *27F1-2 | 0.25 µl |
| 100 µM *27R1-2 | 0.25 µl |
| 5 U/µl GeneTaq NT (trade name, NIPPON GENE CO., LTD.) | 0.25 µl |
| | 49 µl |

*3FL-27-wt-F3-27: 5'-gtttattcccCgtatgcaacccttgcc-(BODIPY FL) [SEQ ID NO: 7]
*27F1-2: 5'-agaactttctgtgcgacg [SEQ ID NO: 8]
*27R1-2: 5'-cagatgcagagctcaatagg [SEQ ID NO: 9]

FIG. 1 is a graph showing the change in fluorescence intensity with an increase in the number of cycles of the PCR. As shown in FIG. 1, the presence of nucleic acids in the sample was verified in both Examples 7-1 and 7-2 from the fact that the fluorescence intensity decreased after performing a certain number of cycles. That is, it can be said that, with the use of the magnetic silica particles having a particle diameter of 6 $\mu$m or less and a specific surface area of 50 m$^2$/g or less, leukocytes in blood can be collected and nucleic acids can be collected from the leukocytes.

Furthermore, from the fact that the fluorescence intensity obtained in Example 7-1 was lower than that in Example 7-2, it can be said that a larger amount of nucleic acids could be detected (collected) in Example 7-2 than in Example 7-1. That is, it can be said that, when collecting leukocytes in blood, nucleic acids can be collected still more efficiently by using a neutral solution containing MgCl$_2$ or the like as a microorganism collection solution.

Example 8

The present example is another example where cells (leukocytes) were collected using magnetic silica particles, and nucleic acids were collected from the cells.

Example 8-1 was the same as Example 7, except that magnetic silica particles having a particle diameter of 0.1 to 0.4 $\mu$m and a specific surface area of 2 to 10 m$^2$/g (trade name S-M-05, BANDO CHEMICAL INDUSTRIES, LTD.) were used, 0.5 M Tris-HCl (pH 7.2) containing 1M MgC1$_2$ was used as a buffer, 10 mM Tris-HCl (pH 8) containing 0.1 mM EDTA (pH 8) was used as a cleaning solution, and PCR amplification was performed by heating extracted nucleic acids for 2 minutes and at 95°C for 2 minutes, followed by 50 cycles each of which consisted of 95°C for 15 seconds and 56°C for 45 seconds, using reagents shown in Table 13 below.

Example 8-2 is the same as Example 8-1, except that magnetic silica particles having a particle diameter of 2 $\mu$m and a specific surface area of 2.7 m$^2$/g (trade name Micromer (R)-M, Micromod) were used. Also, Comparative Examples 6-1 and 6-2 were the same as Examples 8-1 and 8-2, respectively, except that magnetic silica particles having a particle diameter of 12 $\mu$m and a specific surface area of 0.45 m$^2$/g (Micromer (R)-M, Micromod) were used.

[Table 13]

| | |
|---|---|
| H$_2$O | 19.2 $\mu$l |
| 10 × GeneTaq Buffer (trade name, NIPPON GENE CO., LTD.) | 2.5 $\mu$l |
| 10 mM AUGC | 0.5 $\mu$l |
| 100 mM MgCl$_2$ | 0.37 $\mu$l |
| 5 $\mu$M F-T-IAPP-F1-wt-34* | 1 $\mu$l |
| 100 $\mu$M IAPP F250-267* | 0.125 $\mu$l |
| 100 $\mu$M IAPP R354-331* | 0.125 $\mu$l |
| 5 U/$\mu$l GeneTaq NT (trade name, NIPPON GENE CO., LTD.) | 0.125 $\mu$l |
| 2 U/$\mu$l UNG (TREVIGEN, Inc) | 0.05 $\mu$l |
| | 24 $\mu$l |

*F-T-IAPP-F1-wt-34: (FAM)-ttcattccagcaacaactttggtgccattctctc-(TAMRA) [SEQ ID NO: 10]
*IAPP F250-267: 5'-cacatgtgcaacgcagcg-3' [SEQ ID NO: 11]
*IAPP R354-331: 5'-ctcttgccatatgtattggatccc-3' [SEQ ID NO: 12]

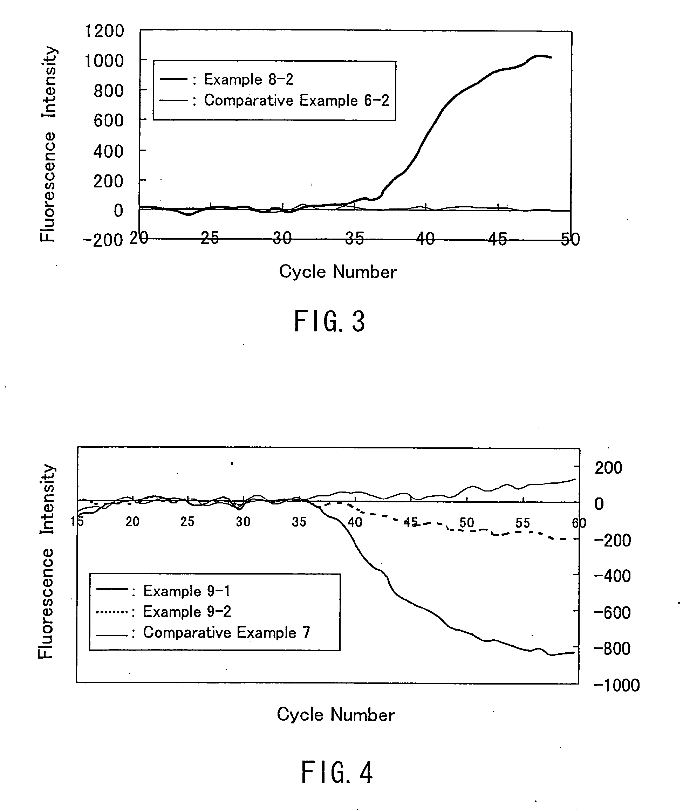

FIG. 2 shows the measurement results of the real-time PCR performed in Example 8-1 and Comparative Example 6-1, and FIG. 3 shows the measurement results of the real-time PCR performed in Example 8-2 and Comparative Example 6-2. As shown in FIG. 2, in Example 8-1 where the fine particles having a particle diameter of 6 $\mu$m or less and a specific surface area of 50 m$^2$/g or less were used, a larger amount of the nucleic acids could be collected as compared with Comparative Example 6-1 where the fine particles having a particle diameter not satisfying the above-described range were used. Furthermore, as shown in FIG. 3, in Example 8-2 where the fine particles having a particle diameter of 6 $\mu$m or less and a specific surface area of 50 m$^2$/g or less were used, a larger amount of the nucleic acids could be collected as compared with Comparative Example 6-2 where the fine particles having a particle diameter not satisfying the above-described range were used.

Example 9

The present example is an example where viruses (HCVs) were collected using magnetic silica particles, and nucleic acids were collected from the thus-collected viruses.

First, magnetic silica particles shown in Table 14 below were provided.
[Table 14]

| | | Particle diameter (μm) | Specific surface area (m²/g) |
|---|---|---|---|
| Ex. 9-1 | Trade name: S-M-03 (BANDO CHEMICAL INDUSTRIES, LTD.) | 0.1 to 0.4 | 2 to 10 |
| Ex. 9-2 | Trade name: Micromer(R)-M (Micromod) | 2 | 2.7 |
| Comp. Ex. 7 | Trade name: Micromer(R)-M (Micromod) | 12 | 0.45 |

Then, to 50 μl of HCV Plasma (ProMedDx, Number 9990964), 50 μl of a buffer (0.5 M glycine-citric acid (pH 3), 40% ethanol) and 8 μl of a solution containing any one of the above-noted magnetic silica particles (500 mg/ml) were added. The HCVs were adsorbed onto the magnetic silica particles by subjecting the resultant mixture to pipetting 30 times. The supernatant was removed in the same manner as described above. Thereafter, 200 μl of distilled water (OTSUKA PHARMACEUTICAL CO., LTD.) was added and the resultant mixture was subjected to pipetting 10 times, thereby washing the magnetic silica particles on which the HCV was adsorbed. This washing operation was repeated to a total of three times, and the collection of the magnetic silica particles was then performed in the same manner as described above. Nucleic acids of the HCVs were extracted by adding 70 μl of a nucleic acid-extraction reagent (10 mM Tris-HCl (pH 8), 0.1 mM EDTA (pH 8), and 0.1 wt% SDS) and 5 μl of an RNAsecure Reagent (Ambion) to the thus-collected magnetic silica particles, subjecting the resultant mixture to pipetting 10 times, heating the mixture at 95°C for 5 minutes, and further subjecting the mixture to pipetting 10 times. To 75 μl of an nucleic acid extract thus obtained, 25 μl of 20% Nonidet P-40 (Nacalai Tesque, Inc.) was added, and the resultant mixture was subjected to pipetting 10 times and heated at 60°C for 10 minutes.

Reagents shown in Table 15 below were used with respect to 5 μl of the heated nucleic acid extract, and RNAs of the HCVs were detected by performing a real-time PCR using a quenching probe specific to a target sequence. More specifically, using i-Cycler™ (trade name, Bio-Rad Laboratories), the real-time PCR was performed by heating the nucleic acid extract at 60°C for 30 minute and then at 94°C for 2 minutes, subjecting the nucleic acid extract to 60 cycles each of which consisted of 94°C at 15 seconds and 60°C for 30 seconds, and further heating the nucleic acid extract at 60°C for 7 minutes. The results are shown in FIG. 4.

[Table 15]

| | |
|---|---|
| H₂O | 6.75 μl |
| 5 × **Reaction Buffer | 5 μl |
| 10 mM AUGC | 0.75 μl |
| 100 μM *HC-F303-25-3a | 0.25 μl |
| 100 μM *HCV-R402-28 | 0.125 μl |
| 25 mM **Mn(OAc)₂ | 1 μl |
| 10 U/μl **RNase Inhibitor | 1 μl |
| 2.5 U/μl **rTth DNA polymerase | 1 μl |
| 5 μM *3FL-HCV-347-27 | 1 μl |
| 40% glycerol | 3.125 μl |
| | 20 μl |

*3FL-HCV-347-27: gcagtaccacaaggcctttcgcgaccc-(BODIPY FL) [SEQ ID NO: 13]
*HC-F303-25-3a: 5'-ccccgcgagatcactagccgagtag-3' [SEQ ID NO: 14]
*HCV-R402-28: 5'-gctcatgatgcacggtctacgagacctc-3' [SEQ ID NO: 15]
Note here that, in Table 15, the reagents assigned with the mark "**" are contained in a product available under the trade name RT-PCR high-Plus-<<GYAKUTEN IPPATSU>> (TOYOBO CO., LTD).

FIG. 4 is a graph showing the change in fluorescence intensity with the number of cycles of the PCR. As shown in FIG. 4, in Examples (9-1 and 9-2) where the fine particles having a particle diameter of 6 μm or less and a specific surface area of 50 m²/g or less were used, quenching was observed at an early stage of the cycles, which demonstrates that the amount of the nucleic acids collected was large. On the other hand, in Comparative Example 7 where the fine particles having a particle diameter not satisfying the above-described range were used, quenching hardly was observed, which demonstrates that the collection rate was low. From these results, it can be said that, with the use of fine particles having a particle diameter of 6 $\mu$m or less and a specific surface area of 50 m$^2$/g or less, it is possible to collect virus efficiently.

Example 10

The present example is another example where viruses (HCVs) were collected using magnetic silica particles, and nucleic acids were collected from the viruses.

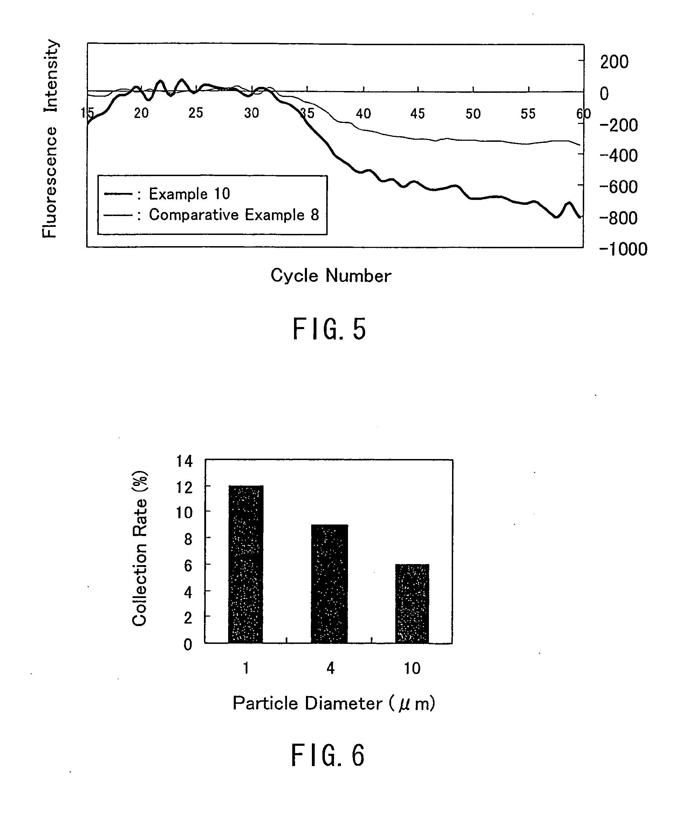

To 50 $\mu$l of HCV Plasma (ProMedDx, Number 9990964), 50 $\mu$l of a buffer (0.5 M glycine-HCl (pH 3) and 40% ethanol) and 10.64 $\mu$l of a solution containing magnetic silica particles (trade name: S-M-04; BANDO CHEMICAL INDUSTRIES, LTD., particle diameter: 0.1 to 0.4 $\mu$m, specific surface area: 2 to 10 m$^2$/g) (376 mg/ml) were added. HCVs were adsorbed onto the magnetic silica particles by subjecting the resultant mixture to pipetting 30 times. A supernatant was removed in the same manner as described above, and 100 $\mu$l of distilled water (OTSUKA PHARMACEUTICAL CO., LTD.) was added and the resultant mixture was subjected to pipetting 10 times. In this manner, the magnetic silica particles on which the HCVs were adsorbed were washed. This operation was repeated to a total of three times. A supernatant was removed in the same manner as described above, and nucleic acids of the HCV were extracted by adding 70 $\mu$l of a nucleic acid-extraction reagent (10 mM Tris-HCl (pH 8), 0.1 mM EDTA (pH 8), 0.1% SDS) and 5 $\mu$l of an RNAsecure Reagent (Ambion) to the thus-collected magnetic silica particles, subjecting the resultant mixture to pipetting 10 times, heating the mixture at 95°C for 5 minutes, and further subjecting the mixture to pipetting 10 times. To 75 $\mu$l of an nucleic acid extract thus obtained, 25 $\mu$l of 20% Nonidet P-40$^{(R)}$ (Nacalai Tesque, Inc.) was added, and the resultant mixture was subjected to pipetting 10 times and heated at 60°C for 10 minutes. RNAs of the HCVs were detected by performing a real-time PCR using a quenching probe specific to a target sequence in the same manner as in Example 9. The results are shown in FIG. 5.

(Comparative Example 8)

As Comparative Example 8, RNAs of HCVs were purified from 50 $\mu$l of HCV Plasma (ProMedDx, Number 9990964) using a QIAamp Viral RNA Mini Kit (trade name, QIAGEN), and the RNAs of the HCVs were detected by performing a real-time PCR using a quenching probe specific to a target sequence. The results are shown in FIG. 5 together with the results obtained in Example 10.

FIG. 5 is a graph showing the change in fluorescence intensity with the number of cycles in PCR. As shown in FIG. 5, in Example 10 where the magnetic silica particles having a particle diameter of 6 $\mu$m or less and a specific surface area of 50 m$^2$/g or less were used, quenching was observed at an earlier stage of the cycles and lower fluorescence intensities were obtained as compared with a conventional method using no fine particles (Comparative Example 8). From these results, it can be said that, according to the method of the present invention, viruses can be collected more efficiently than in a conventional methods using no fine particles.

Example 11

The present example is still another example where viruses (HIVs) were collected using magnetic silica particles, and nucleic acids were collected from the viruses.

First, fine particles shown in Table 16 below were provided. Then, to 50 $\mu$l of HIV Plasma (ProMedDx, Number 10439039), 50 $\mu$l of a buffer (0.5 M glycine-citric acid (pH 3), 40% ethanol) and 8 $\mu$l of a solution containing any one of magnetic silica particles shown in Table 16 below (500 mg/ml) were added. The HIVs were adsorbed onto the magnetic silica particles by subjecting the resultant mixture to pipetting 30 times. A supernatant was removed in the same manner as described above, and 200 $\mu$l of distilled water (OTSUKA PHARMACEUTICAL CO., LTD.) was added and the resultant mixture was subjected to pipetting 10 times. In this manner, the magnetic silica particles on which the HIVs were adsorbed were washed. This operation was repeated to a total of three times. A supernatant was removed in the same manner as described above, and nucleic acids of the HIV were extracted by adding 70 $\mu$l of a nucleic acid-extraction reagent (10 mM Tris-HCl (pH 8), 0.1 mM EDTA (pH 8), 0.1% SDS) and 5 $\mu$l of an RNAsecure Reagent to the thus-collected magnetic silica particles, subjecting the resultant mixture to pipetting 10 times, heating the mixture at 95°C for 5 minutes, and further subjecting the mixture to pipetting 10 times. To 75 $\mu$l of an nucleic acid extract thus obtained, 25 $\mu$l of 20% Nonidet P-40$^{(R)}$ (Nacalai Tesque, Inc.) was added, and the resultant mixture was subjected to pipetting 10 times and heated at 60°C for 10 minutes. Amplification and detection were carried out using an AMPLICOR HIV-1 monitor v1.5 (Roche Diagnostics K.K.). The absorbances (450 nm) obtained are shown in Table 16 below.

**EP 1 882 738 A1**

[Table 16]

| | | Particle diameter ($\mu$m) | Specific surface area ($m^2$/g) | absorbance (450 nm) |
|---|---|---|---|---|
| Ex. 11-1 | Trade name: S-M-04 (BANDO CHEMICAL INDUSTRIES, LTD.) | 0.1 to 0.4 | 2 to 10 | 2.174 |
| Ex. 11-2 | Trade name: Micromer(R)-M (Micromod) | 2. | 2.7 | 1.243 |
| Ex. 11-3 | Magnetic silica particles used in Example 1-3 | 2 to 6 | 7.06 | 1.023 |
| Comp. Ex. 9 | Trade name: Micromer(R)-M (Micromod) | 12 | 0.45 | 0.139 |

As shown in Table 16, in Examples (11-1, 11-2, and 11-3) where the fine particles having a particle diameter of 6 $\mu$m or less and a specific surface area of 50 $m^2$/g or less were used, the absorbances (450 nm) obtained were greater than that obtained in Comparative Example 9 where the fine particles having a particle diameter not satisfying the above-described range were used. Thus demonstrates that a larger amount of nucleic acids could be collected in these examples. From these results, it can be said that, with the use of fine particles having a particle diameter of 6 $\mu$m or less and a specific surface area of 50 $m^2$/g or less, it is possible to collect viruses (HIVs) with a high collection rate.

Example 12

The present example is still another example where viruses (HIVs) were collected using magnetic silica particles, and nucleic acids were collected from the viruses.

Nucleic acids were collected and amplified in the same manner as in Example 11-2. The subsequent operations were carried out in the same manner as in Example 11-2, except that the nucleic acid extract was diluted so that the concentration as to prepare a series of dilutions in which the concentrations of the nucleic acid extract were 1, 1/5, and 1/25 of the original and the detection of nucleic acids was performed with respect to these dilutions. The absorbances (450 nm) obtained are shown in Table 17 below.

(Comparative Example 10)

The detection was carried out in the same manner as in Example 12, except that 200 $\mu$l of HIV Plasma (ProMedDx, Number 10439039) was used and that a pretreatment and amplification were carried out using AMPLICOR HIV-1 monitor v1.5 (trade name, Roche Diagnostics K.K.). The absorbances (450 nm) obtained are shown in Table 17 below.
[Table 17]

| Dilution ratio | Example 11 | Comparative example 10 |
|---|---|---|
| $\times$ 1 | 2.65 | 2.494 |
| $\times$ 1/5 | 1.871 | 1.852 |
| $\times$ 1/25 | 0.732 | 0.525 |

As shown in Table 17, in Example 11 where the magnetic silica particles having a particle diameter of 6 $\mu$m or less and a specific surface area of 50 $m^2$/g or less were used, the HIV collecting ability was equivalent or superior to the pretreatment performed using the AMPLICOR HIV-1 monitor v1.5. Therefore, it can be said that the microorganism collection method of the present invention can collect viruses highly efficiently.

17

Example 13

The present example is an example showing the effect of the specific surface area and the particle diameter of fine particles on the collection rate of microorganisms (germs).

First, collection of germs (gonococci) and collection of nucleic acids were performed using plural types of magnetic silica particles, whose particle diameters were different from each other but the total surface areas thereof were set to be the same. Specifically, germs (gonococci) were collected, nucleic acids were extracted, and PCR was performed to calculate the collection rate of the nucleic acids in the same manner as in Example 1, except that magnetic silica particles shown in Table 18 below were used.

Note here that the amount of each type of the fine particles to be used was adjusted based on their specific surface area ($m^2/g$) so that the total surface area became 71 $cm^2$. The collection rates obtained are shown in FIG. 6 and Table 18 below.
[Table 18]

|  |  | Particle diameter ($\mu$m) | Specific surface area ($m^2/g$) | Used amount (mg) | Collection rate of nucleic acids (%) |
|---|---|---|---|---|---|
| Ex. 13-1 | Trade name: S-FHQ-01 | 1 | 17.8 | 0.40 | 12 |
| Ex. 13-2 | Trade name: S-FSQ-01 | 4 | 14.7 | 0.48 | 9 |
| Comp. Ex. 11 | Trade name: S-FLQ-01 | 10 | 6.19 | 1.1 | 6 |
| All the particles are available from BANDO CHEMICAL INDUSTRIES, LTD., and surface-modified with $SiO_2$. | | | | | |

As shown in Table 18, in Examples (13-1 and 13-2) where the magnetic silica particles having a particle diameter of 6 $\mu$m or less and a specific surface area of 50 $m^2/g$ or less were used, the nucleic acids could be collected with a collection rate higher than that in Comparative Example 11 where the magnetic silica particles having a particle diameter of greater than 6 $\mu$m were used, even though the total surface areas were the same between the examples and the comparative example.

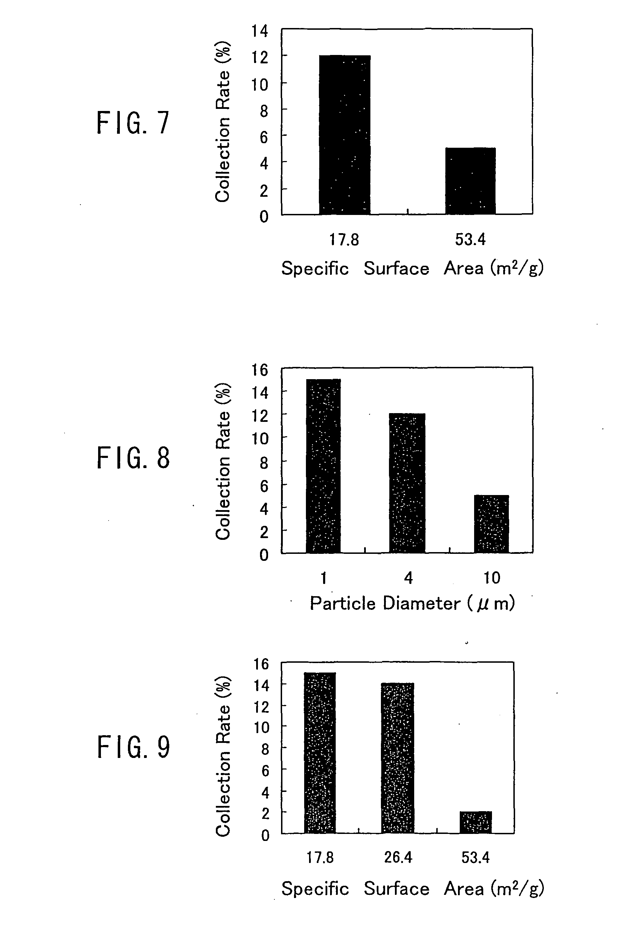

Next, collection of germs (gonococci) and collection of nucleic acids were performed using plural types of magnetic silica particles, whose specific surface areas were different from each other but the particle diameter thereof were the same (1 $\mu$m). Specifically, germs (gonococci) were collected, nucleic acids were extracted, and PCR was performed to calculate the collection rate of the nucleic acids in the same manner as in Example 1, except that magnetic silica particles shown in Table 19 below were used and the amount of the magnetic silica particles used was set to 0.4 mg. The collection rates obtained are shown in FIG. 7 and Table 19 below.
[Table 19]

|  |  | Particle diameter ($\mu$m) | Specific surface area ($m^2/g$) | Collection rate of nucleic acids (%) |
|---|---|---|---|---|
| Ex. 13-3 | Trade name: S-FHQ-01 | 1 | 17.8 | 12 |
| Comp. Ex.12 | Trade name: S-FHQ-01-50 | 1 | 53.4 | 5 |
| All the particles are available from BANDO CHEMICAL INDUSTRIES, LTD., and surface-modified with $SiO_2$. | | | | |

As shown in Table 19, in Example 13-3 where the magnetic silica particles having a specific surface area of 50 $m^2/g$ or less were used, the nucleic acids could be collected with a collection rate higher than that in Comparative Example 12 where the magnetic silica particles having a specific surface area of greater than 50 $m^2/g$ were used.
These results demonstrate that both the particle diameter and the specific surface area of fine particles are important in collection of microorganisms.

Example 14

The present example is an example showing the effect of the specific surface area and the particle diameter of fine particles on the collection rate of microorganisms (viruses).

First, collections of viruses (HBVs) and nucleic acids were performed using plural types of magnetic silica particles, whose particle diameters were different from each other but the total surface areas thereof were set to be the same. Specifically, viruses (HBVs) were collected, nucleic acids were extracted, and PCR was performed to calculate the collection rate of the nucleic acids in the same manner as in Example 6, except that magnetic silica particles shown in Table 20 below were used. Note here that the amount of each type of the fine particles to be used was adjusted based on their specific surface area ($m^2/g$) so that the total surface area became 710 $cm^2$. The collection rates obtained are shown in FIG. 8 and Table 20 below.

[Table 20]

| | | Particle diameter ($\mu$m) | Specific surface area ($m^2/g$) | Used amount (mg) | Collection rate of nucleic acids (%) |
|---|---|---|---|---|---|
| Ex. 14-1 | Trade name: S-FHQ-01 | 1 | 17.8 | 4.0 | 15 |
| Ex.14-2 | Trade name: S-FSQ-01 | 4 | 14.7 | 4.8 | 12 |
| Comp. Ex. 13 | Trade name: S-FLQ-01 | 10 | 6.19 | 11.5 | 5 |
| All the particles are available from BANDO CHEMICAL INDUSTRIES, LTD., and surface-modified with $SiO_2$. | | | | | |

As shown in Table 20, in Examples (14-1 and 14-2) where the magnetic silica particles having a particle diameter of 6 $\mu$m or less and a specific surface area of 50 $m^2/g$ or less were used, the nucleic acids could be collected with a collection rate higher than that in Comparative Example 13 where the magnetic silica particles having a particle diameter of greater than 6 $\mu$m were used, even though the total surface areas were the same between the examples and the comparative example.

Next, collections of viruses (HBVs) and nucleic acids were performed using plural types of magnetic silica particles, whose specific surface areas were different from each other but the particle diameter thereof were the same (1 $\mu$m). Specifically, viruses (HBVs) were collected, nucleic acids were extracted, and PCR was performed to calculate the collection rate of the nucleic acids in the same manner as in Example 6, except that magnetic silica particles shown in Table 21 below were used and the amount of the magnetic silica particles used was set to 4.0 mg. The collection rates obtained are shown in FIG. 9 and Table 21 below.

[Table 21]

| | | Particle diameter ($\mu$m) | Specific surface area ($m^2/g$) | Collection rate of nucleic acids (%) |
|---|---|---|---|---|
| Ex. 14-3 | Trade name: S-FHQ-01 | 1 | 17.8 | 15 |
| Ex. 14-4 | Trade name: S-FHQ-01-25 | 1 | 26.4 | 14 |
| Comp. Ex. 14 | Trade name: S-FHQ-01-50 | 1 | 53.4 | 2 |
| All the particles are available from BANDO CHEMICAL INDUSTRIES, LTD., and surface-modified with $SiO_2$. | | | | |

As shown in Table 21, in Examples (14-3 and 14-4) where the magnetic silica particles having a specific surface area of 50 $m^2/g$ or less were used, the nucleic acids could be collected with a collection rate higher than that in Comparative Example 14 where the magnetic silica particles having a specific surface area of greater than 50 $m^2/g$ were used.

These results demonstrate that both the particle diameter and the specific surface area of fine particles are important in collection of microorganisms.

Industrial Applicability

As specifically described above, according to the microorganism collection method of the present invention, microorganisms can be collected efficiently because fine particles having a particle diameter of 6 $\mu$m or less and a specific surface area of 50 m$^2$/g or less are used. Furthermore, in the nucleic acid collection method of the present invention, microorganisms are collected by the microorganism collection method of the present invention. Therefore, the microorganisms can be collected efficiently, thus allowing nucleic acids to be collected efficiently Accordingly, the present invention is applicable to all the fields where collections of microorganisms, nucleic acids, etc. are required, and can be used suitably for extraction of components from biological samples, for example. However, the present invention can be used for a wide range of applications without limitation.

Sequence Listing Free Text

[SEQ ID NO: 1] F-D-NG-R1-32: TaqMan probe
[SEQ ID NO: 2] NG-F3405-20: oligonucleotide probe designed for forward primers
[SEQ ID NO: 3] NG-3526-20R: oligonucleotide probe designed for reverse primers
[SEQ ID NO: 4] Taqman-B-F-1-32: TaqMan probe
[SEQ ID NO: 5] HBV F50-74: oligonucleotide probe designed for forward primers
[SEQ ID NO: 6] HBV R136-109: oligonucleotide probe designed for reverse primers
[SEQ ID NO: 7] 3FL-27-wt-F3-27: probe
[SEQ ID NO: 8] 27F1-2: oligonucleotide probe designed for forward primers
[SEQ ID NO: 9] 27R1-2: oligonucleotide probe designed for reverse primers
[SEQ ID NO: 10] F-T-IAPP-F1-wt-34: probe
[SEQ ID NO: 11] IAPP F250-267: oligonucleotide probe designed for forward primers
[SEQ ID NO: 12] IAPP R354-331: oligonucleotide probe designed for reverse primers
[SEQ ID NO: 13] 3FL-HCV-347-27: probe
[SEQ ID NO: 14] HC-F303-25-3a: oligonucleotide probe designed for forward primers
[SEQ ID NO: 15] HCV-R402-28: oligonucleotide probe designed for reverse primers

**Claims**

1. A method of collecting microorganisms using fine particles, the method comprising a microorganism adsorption step of bringing a sample into contact with fine particles so as to cause microorganisms contained in the sample to be adsorbed onto the fine particles,
wherein the fine particles have a particle diameter of 6 $\mu$m or less and a specific surface area of 50 m$^2$/g or less.

2. The method according to claim 1, wherein the fine particles have a hydroxyl group on their surfaces.

3. The method according to claim 1, wherein the fine particles are silica particles.

4. The method according to claim 1, wherein the fine particles are magnetic particles.

5. The method according to claim 1, wherein the fine particles are magnetic silica particles.

6. The method according to claim 5, wherein the fine particles are magnetic silica particles containing at least one of a magnetic metal and a magnetic metal oxide.

7. The method according to claim 1, wherein a microorganism collection solution containing the fine particles is provided, and the sample is brought into contact with the microorganism collection solution in the microorganism adsorption step.

8. The method according to claim 7, wherein the microorganism collection solution is an acid solution having a pH of 2 to 3 or a neutral solution containing at least one salt selected from the group consisting of magnesium salts, sodium salts, calcium salts, and potassium salts.

9. The method according to claim 7, wherein the microorganism collection solution is a neutral solution containing a magnesium salt.

10. The method according to claim 7, wherein the microorganism collection solution contains a protein denaturant.

11. The method according to claim 10, wherein the protein denaturant is at least one selected from the group consisting of guanidium salts, ethanol, and acetic acid.

12. The method according to claim 1, wherein the microorganisms are at least one kind selected from the group consisting of germs, viruses and cells.

13. The method according to claim 1, wherein the microorganisms are germs or cells, and the fine particles have a particle diameter of 6 $\mu$m or less and a specific surface area of 20 m$^2$/g or less.

14. The method according to claim 1, wherein the microorganisms are viruses, and the fine particles have a particle diameter of 2 $\mu$m or less and a specific surface area of 30 m$^2$/g or less.

15. A method of collecting nucleic acids, the method comprising:

a microorganism adsorption step of causing microorganisms to be adsorbed onto fine particles; and
a nucleic acid elution step of eluting nucleic acids from the microorganisms that have been adsorbed onto the fine particles,
wherein the microorganism adsorption step is the method according to claim 1.

16. The method according to claim 15, wherein, in the nucleic acid elution step, the nucleic acids are eluted from the microorganisms by bringing the fine particles on which the microorganisms have been adsorbed into contact with a nucleic acid-extraction reagent.

17. The method according to claim 15, further comprising a liquid separation step,
wherein the sample containing the microorganisms is brought into contact with the microorganism collection solution containing the fine particles in the microorganism adsorption step, and
the fine particles on which the microorganisms have been adsorbed and the liquid are separated in the liquid separation step.

18. A kit for collecting microorganisms to be used in the method according to claim 1, the kit comprising:

fine particles having a particle diameter of 6 $\mu$m or less and a specific surface area of 50 m$^2$/g or less.

19. The kit according to claim 18, wherein the fine particles are magnetic silica particles.

20. The kit according to claim 18, further comprising a microorganism collection solution,
wherein the microorganism collection solution contains the fine particles.

21. The kit according to claim 20, wherein the microorganism collection solution is an acid solution having a pH of 2 to 3 or a neutral solution containing at least one salt selected from the group consisting of magnesium salts, sodium salts, calcium salts, and potassium salts.

22. A kit for collecting nucleic acids to be used in the method according to claim 15, the kit comprising:

fine particles having a particle diameter of 6 $\mu$m or less and a specific surface area of 50 m$^2$/g or less; and
a nucleic acid-extraction reagent.

23. The kit according to claim 22, wherein the fine particles are magnetic silica particles.

24. The kit according to claim 22, further comprising a microorganism collection solution,
wherein the microorganism collection solution contains the fine particles.

25. The kit according to claim 24, wherein the microorganism collection solution is an acid solution having a pH of 2 to 3 or a neutral solution containing at least one salt selected from the group consisting of magnesium salts, sodium salts, calcium salts, and potassium salts.

FIG. 1

FIG. 2

FIG. 3

FIG. 4

FIG. 5

FIG. 6

FIG. 7

FIG. 8

FIG. 9

<table>
<tr><td colspan="2" align="center">**INTERNATIONAL SEARCH REPORT**</td><td>International application No.<br>PCT/JP2006/310043</td></tr>
</table>

A. CLASSIFICATION OF SUBJECT MATTER
*C12N15/09*(2006.01), *C12N1/02*(2006.01), *C12N7/02*(2006.01), *C12N11/14*
(2006.01)

According to International Patent Classification (IPC) or to both national classification and IPC

B. FIELDS SEARCHED

Minimum documentation searched (classification system followed by classification symbols)
*C12N15/09*(2006.01), *C12N1/02*(2006.01), *C12N7/02*(2006.01), *C12N11/14*
(2006.01)

Documentation searched other than minimum documentation to the extent that such documents are included in the fields searched
Jitsuyo Shinan Koho          1922-1996   Jitsuyo Shinan Toroku Koho   1996-2006
Kokai Jitsuyo Shinan Koho    1971-2006   Toroku Jitsuyo Shinan Koho   1994-2006

Electronic data base consulted during the international search (name of data base and, where practicable, search terms used)
JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| Y | JP 2002-165591 A  (JSR Corp.),<br>11 June, 2002 (11.06.02),<br>(Family: none) | 1-25 |
| Y | JP 63-028392 A  (Fuji-Davison Chemical Ltd.),<br>06 February, 1988 (06.02.88),<br>(Family: none) | 1-25 |
| Y | JP 62-269691 A  (ROHM & HAAS CO.),<br>24 November, 1987 (24.11.87),<br>& EP 245986 A          & US 4952506 A | 1-25 |
| Y | JP 2003-265164 A  (KANEKA Corp.),<br>24 September, 2003 (24.09.03),<br>(Family: none) | 1-25 |

☐ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>05 June, 2006 (05.06.06) | Date of mailing of the international search report<br>13 June, 2006 (13.06.06) |
|---|---|
| Name and mailing address of the ISA/<br>Japanese Patent Office | Authorized officer |
| Facsimile No. | Telephone No. |

Form PCT/ISA/210 (second sheet) (April 2005)

**EP 1 882 738 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003521228 A
- JP 2002507116 A